# FASCICULE DE BREVET EUROPEEN

(11) **EP 0 311 528 B1**
(45) Date de publication et mention de la délivrance du brevet: **01.06.1994**
(21) Numéro de dépôt: 88402545.3
(22) Date de dépôt: 07.10.1988
(51) Int. Cl.: A61B 17/06

(54) **Emballage pour ligatures et sutures chirurgicales sous la forme d'un étui**
Packung für chirurgisches Nahtmaterial und Abbindungen in der Form eines Etuis
Case shaped package for surgical sutures and ligatures

(30) Priorité: 09.10.1987 FR 8713997
(43) Date de publication de la demande: 12.04.1989
(73) Titulaire: PETERS, 93000 Bobigny (FR)
(72) Inventeur: Schwarz, Georg Erwin, F-95190 Goussainville (FR)
(74) Mandataire: Phélip, Bruno

(56) Documents cités:
- EP-A- 0 128 022
- EP-A- 0 168 161
- CH-A- 435 566
- CH-A- 526 965
- GB-A- 1 062 696
- US-A- 3 298 559

## Description

La présente invention concerne un ensemble de présentation d'un fil de suture chirurgicale et/ou d'une aiguille dans un emballage pour ligatures et sutures chirurgicales sous la forme d'un étui constitué par au moins deux panneaux. L'invention concerne également un procédé de mise en forme d'un tel ensemble de présentation.

Depuis l'utilisation des ligatures et des sutures en chirurgie, les fabricants de ces articles ont essayé de trouver le support le plus pratique et le moins traumatisant pour les fils desdites ligatures et sutures.

Il faut que le fil, avec ou sans aiguilles, puisse être rapidement enlevé de l'emballage et du support pendant l'opération, qu'il ne s'emmêle pas et qu'il ne garde pas l'empreinte due au transport.

Dans l'art antérieur, on a utilisé des supports en verre, en plastique moulé ou à alvéoles et en papier, sous forme de bobines, de navettes, de plaques ou d'étuis repliés. On a proposé une multitude d'emballages.

Ainsi plus particulièrement, FR-A-78.25.117 décrit et illustre un empaquetage à panneaux multiples comportant des arrêtoirs tridimensionnels pour fixer la boucle du fil ou l'aiguille.

Dans US-A-4.369.880, on a décrit un emballage pour suture, où l'aiguille est fixée dans une poche aménagée et, en poussant le bas de l'emballage, l'aiguille se soulève.

US-A-4.412.614 a pour objet un emballage à plusieurs compartiments, dans lesquels sont aménagées des ouvertures laissant passage à des clavettes pour l'enroulement du fil. De plus, la configuration des panneaux est telle que les panneaux intérieurs et l'emballage extérieur se soudent et s'ouvrent ensemble.

Les ouvertures pour l'enroulement du fil sont cachées par des panneaux supplémentaires selon le brevet US-A.4.427.109.

D'autres variantes de support avec une matière à alvéole sur du papier cartonné ou d'autres matières sont connues et décrites dans US-A-4.412.613, US-A-4.406.363, et US-A-4.413.727.

Les aiguilles de ces sutures sont piquées et fixées dans une mousse alvéolée qui les maintient.

Tous ces différents supports présentent généralement un emballage intérieur de distribution et sont toujours conditionnés dans un ou plusieurs emballages, faisant fonction de barrière antimicrobienne préservant ainsi la stérilité du fil de suture.

Il existe aussi des boîtiers ou pochettes où l'emballage intérieur et l'emballage extérieur s'ouvrent en même temps, permettant une distribution directe du fil ou de l'aiguille. Ceci peut présenter un gain de temps mais aussi des problèmes d'asepsie. Cette technique est décrite dans FR-A-78.07.706 et dans US-A-4.412.614.

CH-A-526.965 décrit un emballage extérieur en complexe d'aluminium-polyéthylène-papier qui assure l'étanchéité à la vapeur d'eau et à l'oxygène et protège ainsi la ligature qui est à l'intérieur de la pochette. Cette ligature est à base d'acide polyglycolique qui est très sensible à l'humidité et qui se décomposerait en présence de vapeur d'eau.

GB-A-1.062.696 décrit et illustre un emballage pour sutures à base d'aluminium ou complexe d'aluminium étanche afin de maintenir l'alcool à l'intérieur de cette petite pochette ainsi formée, d'éviter son évaporation et de pouvoir mettre cette pochette dans un liquide désinfectant en évitant ainsi que le liquide désinfectant rentre de l'extérieur vers l'intérieur.

US-A-3 298 559 décrit et illustre des récipients formés à froid constitués de stratifié plastique métal.

EP-A-0 128 022 concerne un dispositif de retenue de sutures à plusieurs compartiments permettant une meilleure protection pendant la stérilisation et le conditionnment subséquent.

La présente invention est definie par les revendications indépendantes 1 et 13. Elle propose en particulier un emballage qui permet un enlèvement rapide du fil, dans lequel le fil ne s'emmêle pas et ne garde pas l'empreinte due au support.

La présente invention n'utilise ni trous de passage ou ouvertures pour l'enroulement du fil, qui peuvent créer des problèmes pour des fils sensibles à la chaleur pendant la stérilisation ou pendant l'emballage (voir US-A4 427 109 précité) ou des problèmes d'asepsie, ni poche ou mousse alvéolée pour fixer et maintenir en place l'aiguille de la suture qui peut se décoller, s'effriter ou se détériorer à la longue.

La présente invention a pour objet un emballage pour ligatures et sutures chirurgicales sous la forme d'un étui constitué d'au moins deux panneaux, qui est caractérisé en ce qu'il comprend une mince feuille métallique déformable, assurant la mise en place et le maintien du fil de suture ou de l'aiguille par leur empreinte dans ladite feuille métallique.

La présente invention concerne également les caractéristiques ci-après considérées isolément ou selon toutes leurs combinaisons techniquement possibles:
- la feuille mince métallique constitue la partie interne ou externe de l'étui;
- la partie externe forme un complexe avec la partie interne;
- la partie externe et/ou interne est en papier;
- la partie externe et/ou interne est en tissu;
- la partie externe et/ou interne est en non tissé;
- la partie externe et/ou interne est en matière plastique;
- la partie externe et/ou interne est formée d'une feuille métallique analogue;
- le complexe est formé d'une feuille métallique d'une part et de papier, de tissu, de non tissé ou de matière plastique d'autre part, ou d'une combinaison de plusieurs feuille de ces matériaux entre eux dans un ordre quelconque, la partie métallique pouvant se trouver indifféremment à l'intérieur ou à l'extérieur de l'étui ou à l'intérieur du complexe;
- la partie interne a une épaisseur comprise dans la gamme de 10 à 60 microns, tandis que la partie externe a une épaisseur comprise dans la gamme de 5 à 100 microns;
- la feuille métallique est choisie parmi l'aluminium, l'étain, le plomb et leurs alliages compatibles ;
- la feuille métallique est recouverte d'un vernis ou d'une peinture;
- la mince feuille métallique est une feuille lisse plane dépourvue de trous , d'aspérités et d'éléments tridimensionnels;
- la feuille métallique mince est une feuille d'aluminium d'environ 40 microns et la partie externe est du papier à environ 80 g/m²;
- l'emballage comprend trois panneaux et un rabat, le panneau central est relié d'une part à un panneau de forme analogue pourvu d'un évidement pour le passage du fil et d'autre part à un panneau à pan coupé, lequel est relié à un rabat pour fermer l'étui ;
- pour garder l'empreinte dans la feuille métallique ou le complexe, on l'emboutit légèrement lors de la mise en place soit manuellement soit avec un moyen mécanique.

Divers avantages et caractéristiques de la présente invention ressortiront de la description détaillée ci-après faite en regard des dessins annexés sur lesquels :
Fig.1 est une vue de face d'un étui de l'invention conditionné dans des suremballages ;
Fig.2 est une vue de face d'un étui déployé selon l'invention ;

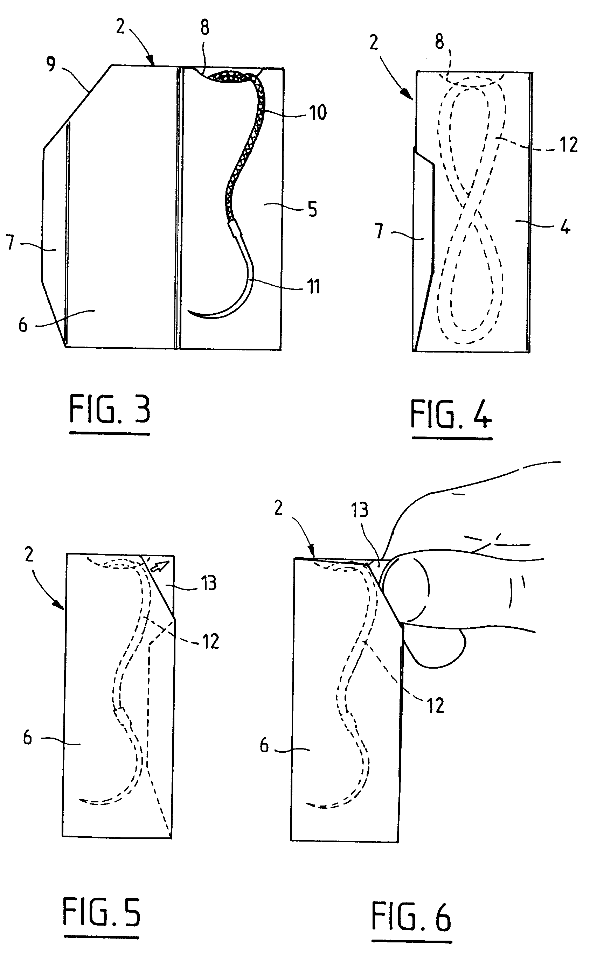

Fig.3 est une vue de face avec un panneau replié;
Fig.4 est une vue postérieure d'un étui fermé ;
Fig.5 est une vue de face d'un étui conditionné ;
Fig.6 est une vue de face en position d'ouverture d'un étui selon l'invention.

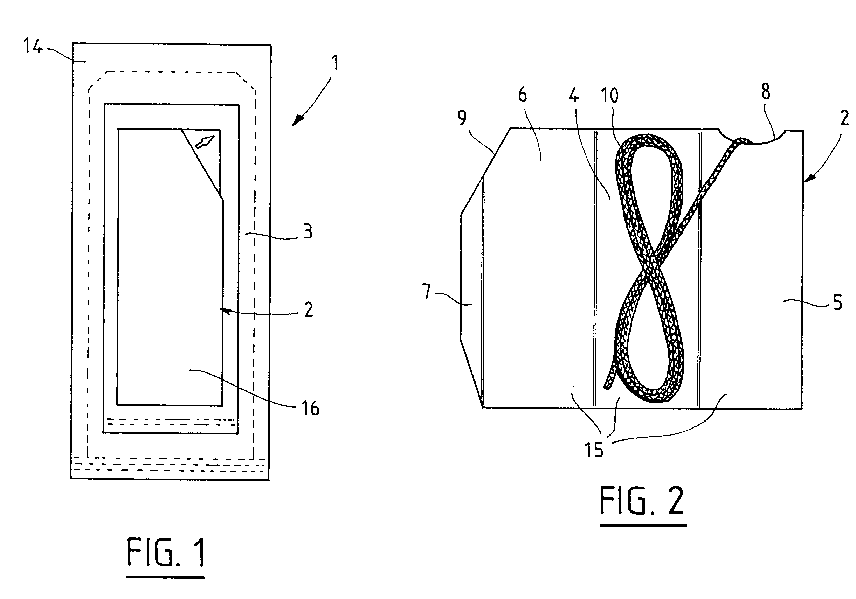

Aux dessins annexés, où les mêmes symboles de références désignent des parties analogues, l'emballage est désigné dans son ensemble par 1. Il comprend un étui 2 constitué de trois panneaux 4, 5 et 6 et d'un rabat 7. Comme montré sur la figure 1, l'étui 2 est disposé dans une enveloppe 3 et cet ensemble se trouve dans un suremballage 14.

Comme illustré sur les figures, l'étui 2 est formé autour du fil 10 est de l'aiguille 11 . Le fil de suture 10 qui est enroulé de façon à éviter qu'il ne s'emmêle lors du déroulement, plus précisément selon une forme de huit , est placé entre deux panneaux 4, 5 d'une feuille de complexe aluminium-papier d'une rigidité telle que le fil 10 soit maintenu par son empreinte 12 dans le complexe. L'aiguille 11 reste également coincée par le panneau 6 maintenu par son empreinte 12. L'étui 2 ainsi formé est maintenu par un rabat 7. Un pan coupé 9 dans le panneau 6 permet au chirurgien ou à l'instrumentiste un dégagement facile de l'aiguille 11 et le fil 10 sortant de son empreinte 12 se dégage instantanément sans s'emmêler par le haut de l'étui 2. L'étui 2 présente ainsi une partie interne 15 et une partie externe 16 formant ensemble complexe.

La rigidité du complexe formant l'étui 2 est une caractéristique importante de l'invention, de même que l'épaisseur de la feuille métallique, notamment de l'aluminium du complexe. Trop mince, elle ne garde ni l'empreinte ni le pliage qu'on lui a donné; trop épaisse, elle écrase le fil et risque de le couper. L'épaisseur de la feuille métallique du complexe doit être comprise dans la gamme de 10 à 60 microns et les meilleurs résultats sont obtenus avec une épaisseur de 30 à 40 microns.

La feuille métallique, le plus souvent en aluminium, peut être revêtue d'une peinture ou d'un vernis, afin de faciliter son impression et permettre ainsi d'y apposer des moyens d'identification ou de marquage.

La feuille mince métallique utilisée dans l'invention à titre de doublure peut être complexée par du papier , du tissu, du non tissé , du polyéthylène, du polypropylène , du polyamide , du polychlorure de vinyle et plus généralement avec toute autre matière plastique conve nable susceptible d'augmenter sa solidité au pli. Il est également possible d'utiliser un complexe ayant plus de deux composants.

Les meilleurs résultats sont obtenus avec un complexe aluminium-papier qui permet de plus d'imprimer l'étui 2 afin d'identifier la suture. L'épaisseur du papier peut être choisie dans la gamme de 10 g/m² à 100 g/m². On préfère une gamme de 80g/m².

L'étui 2 est formé de trois panneaux 4, 5, 6 et d'un rabat 7. Le panneau central 4 est, de préférence, rectangulaire ainsi que le panneau 5. Le panneau 5 comporte un évidement 8 permettant le passage du fil 10 du premier au deuxième compartiment. Le panneau 6 présente , comme on l'a déjà indiqué , un pan coupé 9 . Il est donc de forme pentagonale .Le rabat 7 qui est relié au panneau 6 est un rabat permettant de maintenir l'étui 2 fermé.

La composition du complexe aluminium-papier a été choisie pour que le complexe puisse être empreint par un objet tridimensionnel, tel qu'un fil de suture ou par exemple une aiguille afin de maintenir en place ces objets dans leur empreinte. On obtient cette empreinte facilement par simple pression du pouce et de l'index. Pour garder l'empreinte , on emboutit légèrement lors de la mise en place soit manuellement comme on vient de l'indiquer soit avec tout moyen mécanique convenable.

La composition du complexe de la présente invention permet également au rabat 7 de garder le pli qu'on lui donne pour maintenir l'étui 2 fermé , ce qui contribue ainsi au maintien de la suture ou de la ligature pendant l'emballage , le conditionnement , le transport et le stockage jusqu'au moment de l'utilisation.

Un autre avantage du complexe aluminium-papier suivant la présente invention est dû à sa facilité d'impression. Afin d'identifier la suture ou ligature contenue dans l'étui fermé 2, on peut utiliser le complexe enroulé sur une bobine déjà préimprimée côté papier et prédécoupée , l'imprimer avec une machine à imprimer ou une imprimante et ensuite conditionner immédiatement le fil et/ou l'aiguille en formant l'étui 2.

Cet autre avantage est obtenu en raison de la rigidité avantageuse du complexe, que l'on peut enrouler sur une bobine, alors que le carton et les supports classiques en matière plastique utilisés autrefois ne peuvent être présentés de cette façon.

La feuille métallique est, de préférence, en aluminium, mais on peut aussi utiliser avec succès de l'étain, du plomb ou des alliages compatibles. Dans un mode de réalisation selon la présente invention, tant la feuille métallique interne 15 que la partie externe 16 peuvent être en aluminium. Cependant, dans les autres modes de réalisation, il s'agit de complexes comme cela a été mentionné ci-dessus. Comme représenté sur les dessins, le coin 13 sert à faciliter l'ouverture par déchirage de l'étui 2.

## Revendications

1. Ensemble de présentation d'un fil de suture chirurgicale et/ou d'une aiguille dans un emballage pour ligatures et sutures chirurgicales sous la forme d'un étui (2) constitué par au moins deux panneaux (4, 5, 6) et présentant une partie interne (15) et une partie externe (16), comprenant une mince feuille métallique déformable, caractérisé en ce que la mise en place et le maintien du fil de suture (10) ou de l'aiguille (11) sont assurés par leur empreinte (12) dans ladite feuille métallique.

2. Ensemble de présentation selon la revendication 1, caractérisé en ce que la mince feuille métallique constitue la partie interne ou externe de l'étui (2).

3. Ensemble de présentation selon l'une des revendications 1 ou 2, caractérisé en ce que la partie externe (16) forme un complexe avec la partie interne (15).

4. Ensemble de présentation selon la revendication 3, caractérisé en ce que la partie externe (16) et/ou la partie interne (15) est en papier, en tissu, en non-tissé, en une matière plastique.

5. Ensemble de présentation selon l'une quelconque des revendications 1 à 3, caractérisé en ce que la partie externe (16) est une feuille métallique analogue à celle de la partie interne (15).

6. Ensemble de présentation selon l'une quelconque des revendications 3 à 5, caractérisé en ce que le complexe est formé d'une feuille métallique (15) d'une part et de papier, de tissu, de non tissé ou de matière plastique d'autre part, ou d'une combinaison de plusieurs feuilles de ces matériaux entre eux dans un ordre quelconque, la partie métallique pouvant se trouver indifféremment à l'intérieur ou à l'extérieur de l'étui ou à l'intérieur du complexe.

7. Ensemble de présentation selon l'une quelconque des revendications 1 à 6, caractérisé en ce que la partie interne (15) et/ou la partie externe (16) a une épaisseur comprise dans la gamme de 10 à 60 microns, tandis que la partie externe (16) et/ou la partie interne (15) a une épaisseur comprise dans la gamme de 5 à 100 microns.

8. Ensemble de présentation selon l'une quelconque des revendications 1 à 7, caractérisé en ce que la feuille métallique est choisie parmi l'aluminium, le plomb, l'étain et leurs alliages compatibles.

9. Ensemble de présentation selon l'une quelconque des revendications 1 à 8, caractérisé en ce que la mince feuille métallique est une feuille lisse plane dépourvue de trous, d'aspérités et d'éléments tridimensionnels.

10. Ensemble de présentation selon l'une quelconque des revendications 1 à 9, caractérisé en ce que la feuille métallique est recouverte d'un vernis ou d'une peinture.

11. Ensemble de présentation selon l'une quelconque des revendications 1 à 10, caractérisé en ce que la feuille métallique (15) est une feuille d'aluminium d'environ 40 microns et la partie externe (16) est du papier à environ 80 g/m².

12. Ensemble de présentation selon l'une quelconque des revendications 1 à 11, caractérisé en ce qu'il comprend trois panneaux (4, 5, 6) et un rabat (7), le panneau central (4) étant relié d'une part à un panneau (5) de forme analogue pourvu d'un évidement (8) pour le passage du fil (10) et d'autre part à un panneau (6) à pan coupé (9), lequel est relié à un rabat (7) pour fermer l'étui (2).

13. Procédé de mise en forme d'un ensemble de présentation selon l'une quelconque des revendications 1 à 12, caractérisé en ce que, pour garder l'empreinte (12) dans la feuille métallique ou le complexe, on l'emboutit légèrement lors de la mise en place, soit manuellement soit avec un moyen mécanique.

## Claims

1. Package assembly for a surgical suture thread and/or for a needle in a surgical ligature and suture package according to a casing (2) comprising at least two panels (4,5,6) and having an internal part (15) and an external part (16) comprising a thin metallic sheet liable to be deformed, characterized in that it is provided to place and hold the suture thread (10) or the needle (11) by the print thereof (12) in said metallic sheet.

2. Package assembly according to claim 1, characterized in that the thin metallic sheet constitutes the internal or external part of the casing (2).

3. Package assembly according to one of claims 1 or 2, characterized in that the external part (16) makes a compound member with the internal part (15).

4. Package assembly according to claim 3, characterized in that the external part (16) and/or the internal part (15) is made of paper, fabric, a non woven material and a plastic material.

5. Package assembly according to any one of claims 1 to 3, characterized in that the external part (16) is a metallic sheet identical to those of the internal part (15).

6. Package assembly according to any one of claims 3 to 5, characterized in that the compound member is constituted with a metallic sheet (15) from one hand and with paper, fabric, a non woven material or a plastic material, on the other hand, or any combination of many sheets of such materials, the metallic part being indifferently inside or outside the casing or inside the compound member.

7. Package assembly according to any one of claims 1 to 6, characterized in that the internal part (15) and/or the external part (16) has a thickness between about 10 and about 60 microns, while the external part (16) and/or the internal part (15) has a thickness between about 5 and about 100 microns.

8. Package assembly according to any one of claims 1 to 7, characterized in that the metallic sheet is selected from aluminium, lead, tin and compatible alloys thereof.

9. Package assembly according to any one of claims 1 to 8, characterized in that the thin metallic sheet is a plane smooth foil without holes, asperities and tridimentional members.

10. Package assembly according to any one of claims 1 to 9, characterized in that the metallic sheet is covered with a varnish or a painting.

11. Package assembly according to any one of claims 1 to 10, characterized in that the metallic sheet (15) is an aluminium foil of about 40 microns and the external part (16) is paper about 80 g / m².

12. Package assembly according to any one of claims 1 to 11, characterized in that it comprises three panels (4,5,6) and a flap (7), the central panel (4) being linked, on one hand, to an identical panel (5) provided with a notch (8) for the passage of the thread (10) and on the other hand to a panel (6) with a cut side (9) which is linked to flap (7) for closing the casing (2).

13. Method to put in practice a package assembly according to any one of claims 1 to 12, characterized in that in view to keep the print (12) in the metallic sheet or the compound member, it is slightly embossed to place it either manually or with a mechanical means.

## Patentansprüche

1. Darreichungsanordnung eines chirurgischen Nähfadens und/oder einer Nadel in einer Verpackung für chirugisches Abbinde- und Nahtmaterial in Form eines aus wenigstens zwei Feldern (4, 5, 6) bestehenden und eine Innenseite (15) und eine Außenseite (16) aufweisenden Etuis (2), das eine dünne deformierbare metallische Folie enthält, dadurch gekennzeichnet, daß das Einbringen und die Halterung des Nähfadens (10) oder der Nadel (11) durch ihre Einprägung in die Metallfolie gesichert sind.

2. Darreichungsanordnung nach Anspruch 1, dadurch gekennzeichnet, daß die dünne Metallfolie die Außenseite oder die Innenseite des Etuis (2) bildet.

3. Darreichungsanordnung nach Anspruch 1 oder 2, dadurch gekennzeichnet, daß die Außenseite (16) einen Komplex mit der Innenseite (15) bildet.

4. Darreichungsanordnung nach Anspruch 3, dadurch gekennzeichnet, daß die Außenseite (16) und/oder die Innenseite (15) aus Papier, Gewebe, Faservlies oder Kunststoffmaterial ist.

5. Darreichungsanordnung nach einem der Ansprüche 1 bis 3, dadurch gekennzeichnet, daß die Außenseite (16) eine Metallfolie analog zu der Innenseite (15) ist.

6. Darreichungsanordnung nach einem der Ansprüche 3 bis 5, dadurch gekennzeichnet, daß der Komplex durch eine Metallfolie (15) einerseits und Papier, Gewebe, Faservlies oder Kunststoffmaterial andererseits, oder durch eine Kombination mehrerer Blätter dieser Materialien untereinander in einer beliebigen Reihenfolge gebildet wird, wobei die Metallfolie sich auf der Innen- oder Außenseite des Etuis oder im Innern des Komplexes befinden kann.

7. Darreichungsanordnung nach einem der Ansprüche 1 bis 6, dadurch gekennzeichnet, daß die Innenseite (15) und/oder die Außenseite (16) eine Dicke zwischen 10 und 60 Mikrometern aufweist, während die Außenseite (16) und/oder die Innenseite (15) eine Dicke zwischen 5 und 100 Mikrometern aufweist.

8. Darreichungsanordnung nach einem der Ansprüche 1 bis 7, dadurch gekennzeichnet, daß die metallische Folie aus Aluminium, Blei, Zinn oder ihren jeweiligen Legierungen besteht.

9. Darreichungsanordnung nach einem der Ansprüche 1 bis 8, dadurch gekennzeichnet, daß die dünne metallische Folie eine glatte plane Folie ist, die von Löchern, Unebenheiten und dreidimensionalen Elementen frei ist.

10. Darreichungsanordnung nach einem der Ansprüche 1 bis 9, dadurch gekennzeichnet, daß die dünne metallische Folie mit Firnis oder einem Anstrich überdeckt ist.

11. Darreichungsanordnung nach einem der Ansprüche 1 bis 10, dadurch gekennzeichnet, daß die metallische Folie (15) eine Aluminiumfolie mit einer Dicke von etwa 40 Mikrometern ist und daß die Außenseite (16) aus Papier mit einer Dicke von 80 g/qm besteht.

12. Darreichungsanordnung nach einem der Ansprüche 1 bis 11, dadurch gekennzeichnet, daß sie drei Felder (4, 5, 6) und eine Lasche (12) umfaßt, wobei das zentrale Feld (4) einerseits mit einem Feld (5) von analoger Form verbunden ist, das mit einer Aussparung (8) für die Passage des Fadens (10) versehen ist, und andererseits mit einem Feld (6) mit einer Abschrägung (9) verbunden ist, an welchem die Lasche (7) zum Schließen des Etuis (2) hängt.

13. Verfahren zum Erhalten einer Darreichungsanordnung nach einem der Ansprüche 1 bis 12, dadurch gekennzeichnet, daß man die Folie zum dauerhaften Einbringen der Vertiefung (12) in die metallische Folie oder den Komplex nach dem Einbringen des Inhalts und dem Zusammenfalten des Etuis leicht mit der Hand oder einem mechanischen Mittel zusammendrückt.
